# EUROPEAN PATENT APPLICATION

(11) **EP 3 545 895 A1**
(43) Date of publication of application: **02.10.2019**
(21) Application number: 16922382.3
(22) Date of filing: 30.12.2016
(51) Int. Cl.: A61B 34/20, A61B 34/10, A61B 18/12

(54) **MEDICAL PATH NAVIGATION METHOD AND SYSTEM, AND MEDICAL PATH PLANNING METHOD**

(30) Priority: 23.11.2016 CN 201611042473
(71) Applicant: Changzhou Lunghealth Medtech Company Limited, Jiangsu 213145 (CN)
(72) Inventor: LIU, Hongyi, Changzhou Jiangsu 213145 (CN); MA, Jiajun, Changzhou Jiangsu 213145 (CN)
(74) Representative: Viering, Jentschura & Partner mbB Patent- und Rechtsanwälte
(86) International application number: PCT/CN2016/113824
(87) International publication number: WO 2018/094847

(57) **Abstract**

The present application discloses a medical path navigation method, planning method and system. The navigation method includes: planning a navigation path; delivering an ablation catheter with a localization sensor into an airway leading to a lesion region according to the navigation path; adjusting a position and a direction of the ablation catheter in the airway based on the localization sensor during a moving process of the ablation catheter in the airway until the ablation catheter is navigated to a planned initial ablation region; and adjusting a position and a direction of the ablation catheter in the lesion region based on the localization sensor during a moving process of the ablation catheter in the lesion region until the ablation catheter is navigated to other ablation regions. The embodiments of the present application may accurately navigate the ablation catheter to a lesion position that is not reachable by a bronchoscope, such as positions at the periphery of the lung, so that the ablation catheter is no longer subjected to a reachable range of the bronchoscope.

## Description

### Cross-reference

This application claims priority to China Patent Application No. 2016110424733, filed on November 23, 2016 and entitled "Medical Path Navigation Method, Planning Method and System", which is hereby incorporated by reference in its entirety.

### Technical Field

The present application relates to the medical technical field, and more particularly relates to a medical path navigation method, a planning method and a system.

### Background

Radiofrequency ablation is a minimally invasive in situ treatment technique for tumors, and its principle is that: when a high-frequency alternating current of an electrode is injected into a lesion tissue, ions in the lesion tissue change with a change of a current direction. The temperature of the lesion tissue raises, and the lesion tissue is killed at a certain temperature (generally 60 ° C), thereby finally coagulating and inactivating tumor tissues.

At present, the common radiofrequency ablation is performed by directly inserting an ablation electrode into a tumor through percutaneous puncture under the guidance of an image technology such as ultrasonic or computed tomography (CT). For lung tumors, the adoption of the percutaneous puncture through which the ablation electrode is inserted into the tumor may cause a risk of pneumothorax. Studies have shown that, the incidence of pneumothorax in the percutaneous puncture mode is 20%-40%, and pneumothorax, as a serious adverse reaction, is potentially lethal. A relatively safe method is performed by inserting an ablation catheter with an electrode into a bronchoscope, delivering it to a tumor part of the lung through a natural airway of the lung for radiofrequency ablation, and then determining a position to be ablated through X-ray.

However, the bronchoscope can only reach a limited range of the bronchus and cannot cover the lesions at the periphery of the lungs. Therefore, the ablation catheter guided by the bronchoscope cannot accurately reach the lesions at the periphery of the lungs.

### Summary

Aspects of the present application provide a medical path navigation method, a planning method and a system for accurately navigating an ablation catheter to a lesion position that is not reachable by a bronchoscope, such as lesion positions at the periphery of the lung, so that the ablation catheter is no longer subjected to a reachable range of the bronchoscope.

There is provided a medical path navigation method in an embodiment of the present application, including:
planning a navigation path to an initial ablation region on a computed tomography (CT) image of a diseased organ or a 3D reconstruction of the CT scan data, wherein the initial ablation region belongs to one of at least one ablation region included in a lesion region;
delivering an ablation catheter with a localization sensor into an airway leading to the lesion region according to the navigation path;
locating an actual position of the ablation catheter in the airway based on the localization sensor during a moving process of the ablation catheter in the airway, and adjusting a position and a direction of the ablation catheter in the airway according to a deviation relation between the actual position of the ablation catheter in the airway and the navigation path until the ablation catheter is navigated to the initial ablation region; and
locating an actual position of the ablation catheter in the lesion region based on the localization sensor during a moving process of the ablation catheter in the lesion region, and adjusting a position and a direction of the ablation catheter in the lesion region according to a deviation relation between the actual position of the ablation catheter in the lesion region and other ablation regions except for the initial ablation region in the at least one ablation region until the ablation catheter is navigated to other ablation regions.

There is further provided a medical path planning method in an embodiment of the present application, including:
importing computed tomography (CT) data of a diseased organ to construct CT images and three-dimensional models;
determining a lesion region on the diseased organ based on the CT images or the three-dimensional models;
dividing the lesion region into at least one ablation region according to a single ablation range of a used ablation catheter;
selecting an initial ablation region from the at least one ablation region; and
planning a navigation path to the initial ablation region on the CT images or the three-dimensional models.

There is further provided a medical path navigation system in an embodiment of the present application, including: a path planning apparatus, a path navigation apparatus and an ablation catheter with a localization sensor.

The path planning apparatus is configured to plan a navigation path to an initial ablation region on a computed tomography (CT) image of a diseased organ or 3D reconstruction of the CT data, wherein the initial ablation region belongs to one of at least one ablation region included in a lesion region.

The ablation catheter is delivered into an airway leading to the lesion region according to the navigation path to ablate the lesion region.

The path navigation apparatus includes: a display module and a navigation module.

The display module is configured to display the CT images, the three-dimensional models and the navigation path.

The navigation module is configured to locate an actual position of the ablation catheter in the airway based on the localization sensor during a moving process of the ablation catheter in the airway, and adjust a position and a direction of the ablation catheter in the airway according to a deviation relation between the actual position of the ablation catheter in the airway and the navigation path until the ablation catheter is navigated to the initial ablation region.

The navigation module is further configured to locate an actual position of the ablation catheter in the lesion region based on the localization sensor during a moving process of the ablation catheter in the lesion region, and adjust a position and a direction of the ablation catheter in the lesion region according to a deviation relation between the actual position of the ablation catheter in the lesion region and other ablation regions except for the initial ablation region in the at least one ablation region until the ablation catheter is navigated to other ablation regions.

In the embodiments of the present application, on the basis of the CT image or the three-dimensional model, the localization sensor of the ablation catheter is used to perform path navigation in an ablation process. The navigation path planned on the basis of the CT image or the three-dimensional model is combined with the localization sensor to locate the ablation catheter to an initial ablation region in the lesion region. Further, in the lesion region, the ablation catheter is located to other ablation regions based on the localization sensor in turn, thereby solving the problem that the ablation catheter cannot be accurately located to a lesion position due to a limitation of the reachable range of the bronchoscope, and accurately navigating the ablation catheter to a lesion position that is not reachable by a bronchoscope, such as lesion positions at the periphery of the lung.

### Brief Description of the Drawings

Drawings described herein are used for providing further understandings of the present application, and constitute one part of the present application. Illustrative embodiments and descriptions thereof in the present application are used for explaining the present application, and do not constitute an improper limitation to the present application. In the drawings:
Fig. 1 is a schematic diagram of a structure of an ablation catheter with a localization sensor provided by an embodiment of the present application;
Fig. 2 is a schematic diagram of a flow of a medical path navigation method provided by another embodiment of the present application;
Fig. 3 is a schematic diagram of a flow of a medical path navigation method provided by another embodiment of the present application;
Fig. 4 is a schematic diagram of a flow of a medical path planning method provided by another embodiment of the present application;
Figs. 5a to 5g are picture processes of a medical path planning method provided by another embodiment of the present application;
Fig. 6 is a schematic diagram of a structure of a medical path navigation system provided by another embodiment of the present application;
Fig. 7 is a schematic diagram of a structure of a medical path navigation system provided by another embodiment of the present application; and
Fig. 8 is a schematic diagram of a structure of a medical path navigation system provided by another embodiment of the present application.

### Detailed Description of the Invention

For the purpose of making objectives, technical solutions and advantages of the present application clearer, clear and complete description will be made below to the technical solutions of the present application in conjunction with specific embodiments and corresponding drawings. Apparently, the described embodiments are merely a part of the embodiments, rather than all the embodiments of the present application. Based on the embodiments of the present application, all other embodiments derived by those ordinarily skilled in the art without creative work shall fall within the protection scope of the present application.

An embodiment of the present application provides a medical path navigation method which is applicable to an ablation catheter with a localization sensor. On the basis of a CT image or a 3D reconstruction of the CT scan data, the medical path navigation method cooperates with the localization sensor of the ablation catheter to perform path navigation in an ablation process, thereby solving the problem that the ablation catheter cannot be accurately located to a lesion position due to a limitation of the reachable range of a bronchoscope, and accurately locating the ablation catheter to a lesion position that is not reachable by a bronchoscope, such as lesion at the periphery of the lungs.

To facilitate understanding of the method provided by the embodiments of the present application, a brief introduction is made at first to the ablation catheter adopted in the embodiments of the present application.

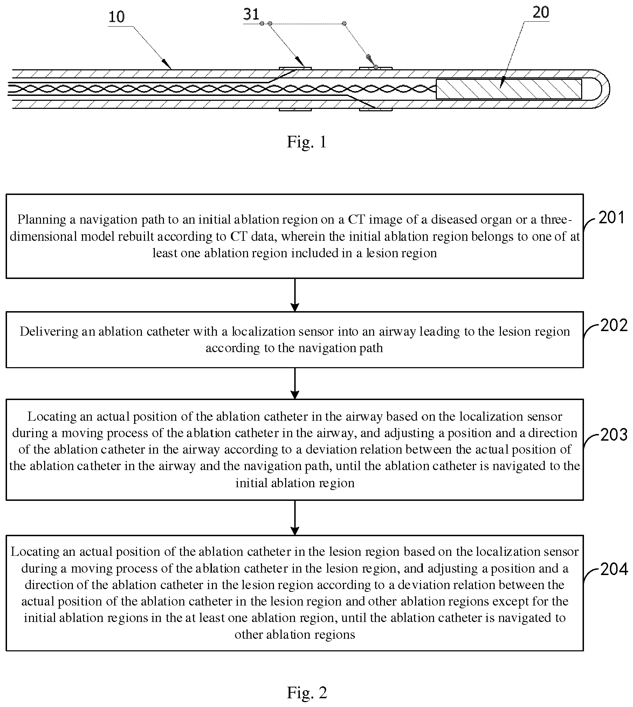

The ablation catheter adopted in the embodiments of the present application is provided with the localization sensor, and has various forms of implementation structures. To facilitate the understanding, Fig. 1 illustrates a structure of an ablation catheter, but it is not limited to this structure. As shown in Fig. 1, the ablation catheter includes: a catheter body 10, a localization sensor 20 and an ablation electrode 31.

The localization sensor 20 is fixedly arranged inside the catheter body 10, and is mainly used for locating the ablation catheter to accurately locate the ablation catheter to a lesion position. For example, the localization sensor 20 may be fixedly arranged inside the catheter body 10 by various methods such as a hot melt method, an adhesive-based adhesion method, and etc.

The localization sensor 20 in the present embodiment adopts an electromagnetic navigation principle which is different from general magnetic navigation principles. The magnetic navigation principle is mainly that: a permanent magnet in the ablation catheter is attracted or repelled by means of an external magnetic field to affect a moving direction of the ablation catheter. The working principle of the localization sensor 20 in the present embodiment is mainly that: a current is output to a control system outside the ablation catheter in response to a magnetic field of a space where the ablation catheter is located, so as to enable the control system to locate the position of the ablation catheter. In detail, the localization sensor 20 is electrically connected with the control system through lead wires. The control system includes a magnetic field generator for generating a magnetic field in a localization space within a certain range. The localization sensor 20 itself is nonmagnetic, and the coil in the localization sensor 20 is used for sensing the magnetic field generated by the magnetic field generator. Herein, the magnetic field generator generates a varying magnetic field in the localization space within the certain range, and ensures that the magnetic field characteristic at each point in the localization space is unique. The coil in the localization sensor 20 generates a current in the varying magnetic field, and the current is transmitted to the control system through the lead wires of the localization sensor 20. The control system converts and analyzes the current transmitted by the localization sensor 20 to determine an accurate position and direction of the ablation catheter.

The ablation electrode 31 is fixedly arranged on the outer surface of the catheter body 10, and is mainly used for performing ablation treatment on a lesion. For example, the ablation electrode 31 may be fixedly arranged on the outer surface of the catheter body 10 by using various methods such as the hot melt method, the adhesion method, a swaging method and a microfluidic technology.

In the present embodiment, the ablation electrode 31 is used for generating a current. The current is injected into a lesion tissue, so that ions in the lesion tissue change with a change of a current direction, the temperature of the lesion tissue raises, and the lesion tissue becomes irreversibly dysfunctional at a certain temperature (generally 60 °C or greater), thereby finally coagulating and inactivating tumor tissues for therapeutic purposes.

Optionally, the ablation catheter of the present embodiment may adopt a unipolar form, namely, the electrode of the catheter body 10 having only one polarity, and the electrode of the other polarity is attached to the outer surface of a human body. For example, the above-mentioned ablation electrode 31 may adopt an annular electrode, and may be made of a metal material, such as copper, stainless steel and a platinum-iridium alloy, or other conductive materials. Alternatively, the ablation catheter of the present embodiment may adopt a bipolar form, namely, the electrodes of the catheter body 10 having positive and negative polarities, and the electrodes of different polarities are not connected, and it is not necessary to use electrode on the outer surface of the human body.

It should be noted that Fig. 1 is only an implementation structure of the ablation catheter, and other structures may be further evolved on the basis of the structure shown in Fig. 1. For example, the ablation catheter may include two or more localization sensors, so as to improve the localization accuracy. For another example, in addition to the localization sensor, the ablation catheter also may include a water-cooling structure used for accommodating electrode cooling liquid, so as to reduce the temperature of the tissue, avoid overheating and scarring, and improve the effectiveness of ablation in the ablation process.

The various ablation catheters provided by the above-mentioned embodiments include the localization sensors in addition to the ablation electrodes. The ablation catheters can be accurately located to the lesion positions through the navigation by the localization sensors. In addition, the positions of the ablation catheters may be further tracked in real time, thereby overcoming the inaccurate positions of the ablation catheters due to various reasons and enhancing the therapeutic effect of the ablation.

On the basis of the ablation catheter as shown in Fig. 1, a medical path navigation method provided by an embodiment of the present application is as shown in Fig. 2, including:
201, a navigation path to an initial ablation region is planned on a computed tomography (CT) image of a diseased organ or a 3D reconstruction of the CT scan data, wherein the initial ablation region belongs to one of at least one ablation region included in a lesion region;
202, an ablation catheter with a localization sensor is delivered into an airway leading to the lesion region according to the navigation path;
203, an actual position of the ablation catheter in the airway is located on the basis of the localization sensor during a moving process of the ablation catheter in the airway, and a position and a direction of the ablation catheter in the airway are adjusted according to a deviation relation between the actual position of the ablation catheter in the airway and the navigation path until the ablation catheter is navigated to the initial ablation region;
204, an actual position of the ablation catheter in the lesion region is located on the basis of the localization sensor during a moving process of the ablation catheter in the lesion region, and a position and a direction of the ablation catheter in the lesion region are adjusted according to a deviation relation between the actual position of the ablation catheter in the lesion region and other ablation regions except for the initial ablation region in the at least one ablation region until the ablation catheter is navigated to other ablation regions.

In the present embodiment, the ablation catheter is required to be navigated to a lesion region of the diseased organ when used for ablation treatment on a diseased organ. The diseased organ mainly refers to the lung, or other organs having a structure similar to the lung. A process of navigating the ablation catheter to the lesion region includes the following stages.

A path planning stage: the CT data of the diseased organ is imported to construct the CT image and the three-dimensional model, and the lesion region on the diseased organ is determined on the basis of the CT image or the three-dimensional model. In the present embodiment, the lesion region may be defined as a single-connected closed three-dimensional region of any shape. For example, it may be a single-connected closed three-dimensional region of a regular shape, such as an ellipsoidal shape; or, the lesion region may also be defined as a simply-connected closed three-dimensional region of any irregular shape.

In consideration that single ablation of the ablation catheter has a certain coverage range, to comprehensively ablate the lesion region, the whole lesion region can be covered through multiple ablations. Based on this consideration, the lesion region may be divided into at least one ablation region. Therefore, one ablation region is ablated each time, and the at least one ablation region is superposed to cover the whole lesion region. Optionally, the ablation region may be spherical, ellipsoidal, sausage shaped or the like. One ablation region is selected as an initial ablation region from the at least one ablation region. For example, one ablation region may be randomly selected as the initial ablation region, or the ablation region located in the center of the lesion region may be selected as the initial ablation region, or the ablation region farthest from a main trachea or a bronchus also may be selected as the initial ablation region. To some extent, the initial ablation region represents the lesion region. The arrival of the ablation catheter to the initial ablation region means the lesion region has been reached. Therefore, the navigation path to the initial ablation region may be planned on the basis of the CT image or the three-dimensional model.

Optionally, in case of more than one ablation region, a path from one ablation region to another ablation region may be further planned until all the ablation regions are reached in turn.

It should be noted that the above-mentioned process of planning the path may be automatically or manually completed.

A first navigation stage: after the navigation path to the initial ablation region is planned, the ablation catheter with the localization sensor is delivered into a natural airway leading to the lesion region according to the navigation path, and the ablation catheter may move in the airway. In order to accurately arrive to a target/lesion region along the planned navigation path, the ablation catheter is navigated in combination with the localization sensor of the ablation catheter on the basis of the navigation path. Specifically, during the moving process of the ablation catheter in the airway, the localization sensor is in a working state, so that the actual position and the direction of the ablation catheter in the airway may be located on the basis of the localization sensor, and the position and the direction of the ablation catheter in the airway are adjusted according to the deviation relation between the actual position of the ablation catheter in the airway and the navigation path planned on the basis of the CT image or the three-dimensional model, until the ablation catheter is navigated to the initial ablation region.

The above-mentioned airway leading to the lesion region includes the main trachea, the bronchus, an inferior bronchus and the like.

In the above-mentioned processes, the localization sensor is electrically connected with a control system outside the ablation catheter. The localization sensor may sense an electromagnetic field at the position of the ablation catheter in the airway to generate an induced current and transmit the induced current to the control system. The control system may calculate the actual position of the ablation catheter in the airway according to the induced current generated by the localization sensor sensing the electromagnetic field at the position of the ablation catheter in the airway, and then adjust the position and the direction of the ablation catheter in the airway according to the deviation relation between the actual position of the ablation catheter in the airway and the navigation path planned on the basis of the CT image or the three-dimensional model until the ablation catheter is navigated to the initial ablation region.

Optionally, the ablation catheter includes two localization sensors, i.e., a first localization sensor and a second localization sensor. The first localization sensor is located at the head portion of the ablation catheter, and the first localization sensor and the second localization sensor are separated by a certain distance. In an application occasion, the two localization sensors of the ablation catheter are in working states simultaneously. Namely, the two localization sensors may both sense the electromagnetic field at the position of the ablation catheter in the airway to generate induced currents and transmit the induced currents to the control system. On the basis of this, in the first navigation stage, the control system may select the first localization sensor, and calculate the actual position of the ablation catheter in the airway according to the induced current generated by the first localization sensor sensing the electromagnetic field at the position of the ablation catheter in the airway. Alternatively, the control system may select the second localization sensor, and calculate the actual position of the ablation catheter in the airway according to the induced current generated by the second localization sensor sensing the electromagnetic field at the position of the ablation catheter in the airway. Alternatively, the control system may select the first localization sensor and the second localization sensor, and calculate the actual position of the ablation catheter in the airway according to the induced currents generated by the first localization sensor and the second localization sensor sensing the electromagnetic field at the position of the ablation catheter in the airway. In another application occasion, one of the two localization sensors is in the working state, so that the actual position of the ablation catheter in the airway may be calculated according to the induced current generated by the localization sensor in the working state through sensing the electromagnetic field at the position of the ablation catheter in the airway.

Optionally, adjusting the position and the direction of the ablation catheter in the airway according to the deviation relation may be, but not limited to, the following methods that: tracking the position and the direction of the ablation catheter in the airway on the basis of the CT image or the three-dimensional model, wherein the navigation path to the initial ablation region may be displayed on the CT image or the three-dimensional model, so that the deviation relation between the actual position of the ablation catheter in the airway and the navigation path can be determined on the basis of the CT image or the three-dimensional model, and the position and the direction of the ablation catheter in the airway can be adjusted on the basis of this deviation relation until the ablation catheter is navigated to the initial ablation region.

Optionally, navigating the ablation catheter to the initial ablation region may be that: navigating an ablation center of the ablation catheter to the center of the initial ablation region, such that an ablation catheter actually ablated region may cover the planned initial ablation region more effectively.

After being navigated to the initial ablation region, the ablation catheter may be controlled to ablate the initial ablation region. If the whole lesion region only includes the initial ablation region, after the ablation catheter is navigated to the initial ablation region, the whole navigation process is ended. If the whole lesion region also includes other ablation regions in addition to the initial ablation region, after the initial ablation region is ablated, other ablation regions are required to be ablated continuously, which means that the ablation catheter is required to be navigated to other ablation regions, i.e., entering a second navigation stage.

The second navigation state: the arrival of the ablation catheter to the initial ablation region means that the ablation catheter is in the lesion region, and if there are other ablation regions, it means that the ablation catheter is required to move from the current ablation region to another ablation region. If a navigation path between the ablation regions is planned in advance, the ablation catheter may move from one ablation region to another ablation region along the planned path. If the navigation path between the ablation regions is not planned in advance, a next ablation region that is required to be arrived to may be randomly selected. During the moving process of the ablation catheter in the lesion region, the localization sensor is in the working state, so that the actual position of the ablation catheter in the lesion region may be located on the basis of the localization sensor, and a moving direction of the ablation catheter in the lesion region is adjusted according to the deviation relation between the actual position of the ablation catheter in the lesion region and other ablation regions until the ablation catheter is navigated to other ablation regions. Optionally, if the navigation path among the ablation regions is planned in advance, in the navigation process, the moving direction of the ablation catheter in the lesion region is adjusted in simultaneously combination with a deviation relation among the actual position of the ablation catheter in the lesion region, the navigation path among the ablation regions, and the centers of the ablation regions, until the ablation catheter is navigated to other ablation regions.

If the number of other ablation regions is one, the ablation catheter may be directly navigated from the initial ablation region to the ablation region. If the number of other ablation regions is multiple, the ablation catheter is required to be navigated for multiple times. The ablation catheter is navigated to one ablation region at each time. After performing the ablation treatment on the ablation region, the ablation catheter is navigated from the ablation region to the next ablation region.

Optionally, the ablation catheter includes two localization sensors, which are respectively a first localization sensor and a second localization sensor. The first localization sensor is located at the head portion of the ablation catheter, and the first localization sensor and the second localization sensor are separated by a certain distance. In an application occasion, the two localization sensors of the ablation catheter are simultaneously in working states. Namely, the two localization sensors may both sense the electromagnetic field at the position of the ablation catheter in the airway to generate induced currents and transmit the induced currents to the control system. On the basis of this, in the second navigation stage, the control system may select the second localization sensor located behind the ablation electrode, and calculate the actual position of the ablation catheter in the airway according to the induced current generated by the second localization sensor sensing the electromagnetic field at the position of the ablation catheter in the airway. As the second localization sensor is located at the rear end of the ablation electrode, it is less interfered by the ablation electrode. The actual position of the ablation catheter in the airway is calculated on the basis of the second localization sensor, which is beneficial to improve the accuracy of a calculation result. Optionally, in order to further reduce electromagnetic interference caused by the ablation electrode, a shielding layer may be further provided for the second localization sensor and lead wires connected with the second localization sensor. In another application occasion, the second localization sensor may be controlled to be in the working state, and the first localization sensor may be controlled to be in a nonworking state. It should be noted that the use of the second localization sensor is preferable, but it is not limited to this.

Optionally, the above-mentioned adjustment of the position and the direction of the ablation catheter in the lesion region according to the deviation relation may adopt, but not limited to, the following method that: the position and the direction of the ablation catheter in the lesion region are tracked on the CT image or the three-dimensional model, wherein positions of other ablation regions may be displayed on the CT image or the three-dimensional model, so that the deviation relations between the position of the ablation catheter in the lesion region and other ablation regions may be determined on the basis of the CT image or the three-dimensional model. Thereupon, the position and the direction of the ablation catheter in the lesion region may be adjusted on the basis of this deviation relation until the ablation catheter is navigated to other ablation regions.

Optionally, navigation of the ablation catheter to other ablation regions may be that: an ablation center of the ablation catheter is navigated to the centers of other ablation regions, so as to enable an ablation catheter actually ablated region may cover other ablation regions more effectively.

It can be seen above that in the embodiments, the CT image or the three-dimensional model is cooperated with the localization sensor of the ablation catheter to perform the path navigation in the ablation process. The ablation catheter may be located to the initial ablation region in the lesion region according to the navigation path planned on the basis of the CT image or the three-dimensional model and in combination with the localization sensor. Further, in the lesion region, the ablation catheter is located to other ablation regions based on the localization sensor in turn, thereby solving the problem that the ablation catheter cannot be accurately located to a lesion position due to a limitation of the reachable range of a bronchoscope, and accurately locating the ablation catheter to a lesion position that is not reachable by a bronchoscope, such as lesion positions at the periphery of the lung, which is no longer subjected to the reachable range of the bronchoscope, and is particularly applicable to some lesion positions that are not reachable by a bronchoscope. In addition, in the navigation process of the present embodiment, X-ray assisted navigation is not required, so that radiation injury caused by the X-ray to a human body may be avoided.

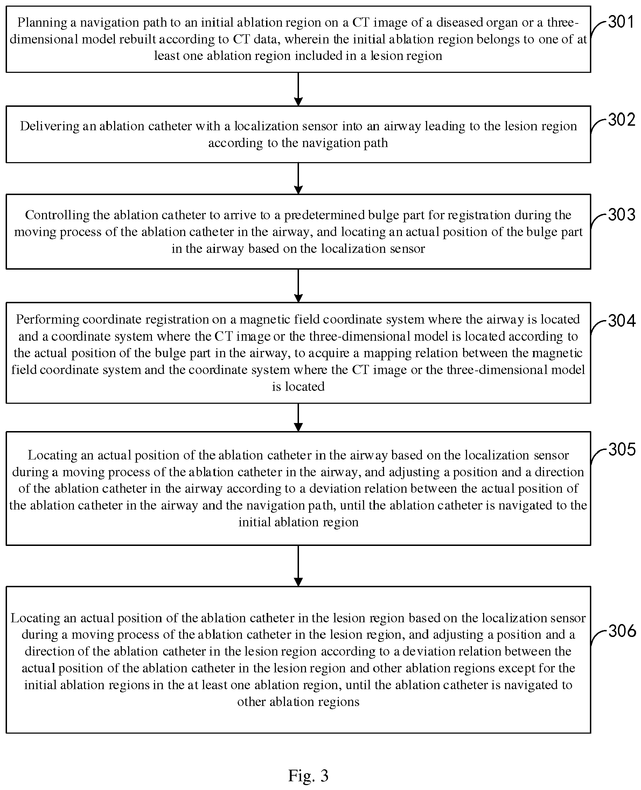

Fig. 3 is a schematic diagram of a flow of a medical path navigation method provided by another embodiment of the present application. This method may be also implemented on the basis of the ablation catheter as shown in Fig. 1. As shown in Fig. 3, the method includes that:
301, a navigation path to an initial ablation region is planned on a computed tomography (CT) image of a diseased organ or a three-dimensional model, wherein the initial ablation region belongs to one of at least one ablation region included in a lesion region;
302, an ablation catheter with a localization sensor is delivered into an airway leading to the lesion region according to the navigation path;
303, the ablation catheter is controlled to arrive to a predetermined carina for registration in a moving process of the ablation catheter in the airway, and an actual position of the carina in the airway is located on the basis of the localization sensor;
304, coordinate registration is performed on a magnetic field coordinate system where the airway is located and a coordinate system where the CT image or the three-dimensional model is located according to the actual position of the carina in the airway, so as to acquire a mapping relation between the magnetic field coordinate system and the coordinate system where the CT image or the three-dimensional model is located;
305, during the moving process of the ablation catheter in the airway, an actual position of the ablation catheter in the airway is located on the basis of the localization sensor, a deviation relation between the actual position of the ablation catheter in the airway and the navigation path is determined on the basis of the mapping relation between the magnetic field coordinate system and the coordinate system where the CT image or the three-dimensional model is located, and a position and a direction of the ablation catheter in the airway are adjusted on the basis of the deviation relation until the ablation catheter is navigated to the initial ablation region;
306, during a moving process of the ablation catheter in the lesion region, an actual position of the ablation catheter in the lesion region is located on the basis of the localization sensor, a deviation relation between the actual position of the ablation catheter in the lesion region and other ablation regions except for the initial ablation region in the at least one ablation region is determined on the basis of the mapping relation between the magnetic field coordinate system and the coordinate system where the CT image or the three-dimensional model is located, and a position and a direction of the ablation catheter in the lesion region are adjusted on the basis of the deviation relation until the ablation catheter is navigated to other ablation regions.

In the present embodiment, the ablation catheter is required to be navigated to a lesion region of the diseased organ when used for ablation treatment on a diseased organ. A process that the ablation catheter is navigated to the lesion region includes several stages: a path planning stage, a registration state, a first navigation stage and a second navigation stage.

The path planning stage in the present embodiment is same as that in the embodiment as shown in Fig. 2, and may refer to the embodiment as shown in Fig. 2, which is not described here in detail.

Based on the navigation path, the ablation catheter is delivered into the airway leading to the lesion region, and may move in the airway. In the present embodiment, after the navigation path is planned, rather than directly entering the first navigation stage, it enters the registration stage. A main objective of the registration is to: register the magnetic field coordinate system where the airway is located and the coordinate system where the CT image or the three-dimensional model is located before navigating the ablation catheter, so as to acquire the mapping relation between the magnetic field coordinate system and the coordinate system where the CT image or the three-dimensional model is located, thereby providing conditions for the subsequent first navigation stage and second navigation stage based on the navigation path, and improving the localization accuracy.

The registration stage includes: a predetermined carina for registration. The present embodiment will not define the number of carinas for registration. For example, there may be 3 or 5 carinas for registration. Preferably, the carinas for registration may include a principal carina, a carina at a next level bifurcation of the left main bronchus and a carina at a next level bifurcation of the right main bronchus, but are not limited to these carinas. Alternatively, it is also possible to perform key points marking on the navigation path, and positions of the corresponding key point marked in the airway are used as key positions for registration. During the moving process of the ablation catheter in the airway, the ablation catheter may be controlled to arrive to the predetermined carina for registration, and the actual position (namely, position coordinates of the carina in the magnetic field coordinate system) of the carina in the airway is located on the basis of the localization sensor. Then, the magnetic field coordinate system where the airway is located and the coordinate system where the CT image or the three-dimensional model is located are registered according to the actual position of the carina in the airway and the position coordinates of a carina mark on the CT image or the three-dimensional model, so as to acquire the mapping relation between the magnetic field coordinate system and the coordinate system where the CT image or the three-dimensional model is located.

Optionally, in the above-mentioned process of arriving to the carina, a bronchoscope may be used for guidance. The carina can be achieved under the assistance of the image of the bronchoscope, and the front end of a current tool (such as the ablation catheter) with the localization sensor touches a selected position on the carina to acquire accurate coordinates of the actual position of the carina.

In the first navigation stage in the present embodiment, during the moving process of the ablation catheter in the airway, the localization sensor generates an induced current by sensing an electromagnetic field at the position of the ablation catheter in the airway and outputs the induced current to a control system. The control system calculates the actual position and the direction, that is, the position coordinates and the orientation of the ablation catheter in an electromagnetic field coordinate system where the airway is located, of the ablation catheter in the airway according to the induced current output by the localization sensor. The actual position of the ablation catheter in the airway is mapped onto the CT image or the three-dimensional model according to the mapping relation between the electromagnetic field coordinate system and the coordinate system where the CT image or the three-dimensional model is located. Herein, the planned navigation path may be displayed on the CT image or the three-dimensional model, so that deviation relations between the position as well as the direction of the ablation catheter and the navigation path may be displayed on the CT image or the three-dimensional model, and then the position and the direction of the ablation catheter in the airway are adjusted on the basis of the deviation relations until the ablation catheter is navigated to the initial ablation region. Other descriptions of the first navigation stage may refer to the embodiment as shown in Fig. 2, and no more details are described here.

In the second navigation stage in the present embodiment, during the moving process of the ablation catheter in the lesion region, the localization sensor generates an induced current by sensing the electromagnetic field at the position of the ablation catheter in the lesion region and outputs the induced current to the control system. The control system calculates the actual position, that is, the position coordinates and the orientation of the ablation catheter in the electromagnetic field coordinate system where the lesion region is located, of the ablation catheter in the lesion region according to the induced current output by the localization sensor. The actual position of the ablation catheter in the lesion region is mapped onto the CT image or the three-dimensional model according to the mapping relation between the electromagnetic field coordinate system and the coordinate system where the CT image or the three-dimensional model is located. Herein, positions of centers of other ablation regions may be displayed on the CT image or the three-dimensional model, so that deviation relations between the position as well as the direction of the ablation catheter and the centers of other ablation regions may be displayed on the CT image or the three-dimensional model, and then the position and the direction of the ablation catheter are adjusted on the basis of the deviation relations until the ablation catheter is navigated to the other ablation regions. Other descriptions of the second navigation stage may refer to the embodiment as shown in Fig. 2, and no more details will be described here.

It can be seen that on the basis of the CT image or the three-dimensional model, the present embodiment may cooperate with the localization sensor of the ablation catheter to perform the path navigation in the ablation process, so that the problem that the ablation catheter may not be accurately located to a lesion position due to a limitation of a reachable range of a bronchoscope may be solved, and the ablation catheter may be accurately located to the lesion position. Furthermore, by coordinate registration, the accuracy of the navigation path may be further improved, thereby improving the localization accuracy.

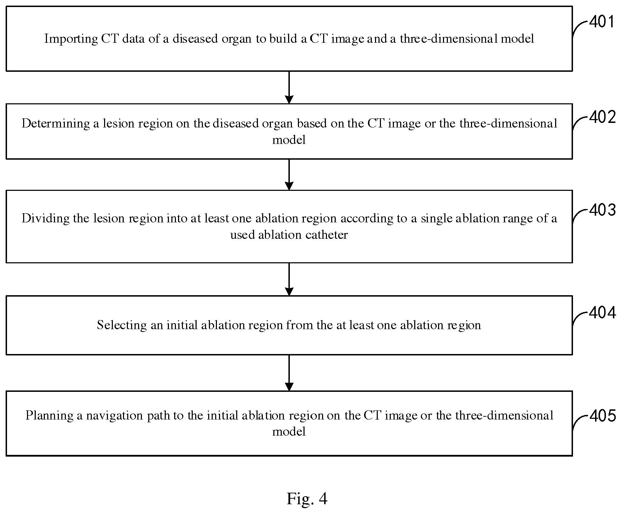

In addition to the above-mentioned medical path navigation method, an embodiment of the present application further provides a medical path planning method. As shown in Fig. 4, the method includes the following steps.

In step 401, computed tomography (CT) data of a diseased organ are imported to construct a CT image and a three-dimensional model.

In step 402, a lesion region on the diseased organ is determined on the basis of the CT image or the three-dimensional model.

In step 403, the lesion region is divided into at least one ablation region according to a single ablation range of a used ablation catheter.

In step 404, an initial ablation region is selected from the at least one ablation region;

In step 405, a navigation path to the initial ablation region is planned on the CT image or the three-dimensional model.

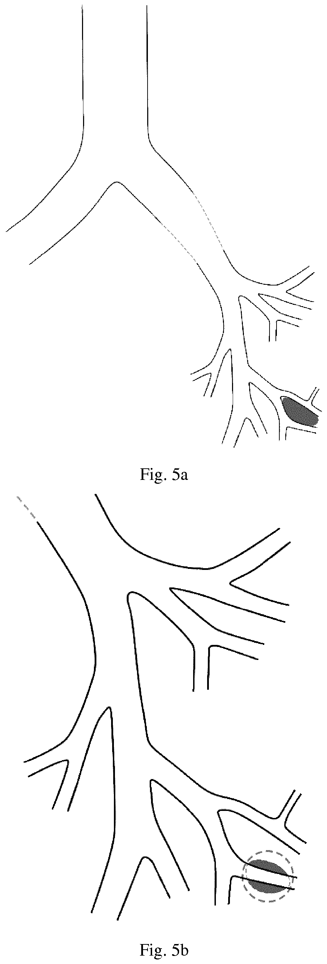

The steps of the above-mentioned method are described in combination with Figs. 5a to 5g.

Firstly, the CT image and the three-dimensional model are built according to the CT data of the diseased organ, and the lesion region on the diseased organ is determined, as shown in Fig. 5a. A shadow region in Fig. 5a is the lesion region. In addition, as shown in Fig. 5a, the lesion region may be a single-connected closed three-dimensional region of any shape; For example, the lesion region may be a regularly shaped single-connected closed three-dimensional region, such as an ellipsoidal shape. Alternatively, the lesion region may also be defined as any irregularly shaped single-connected closed three-dimensional region.

Secondly, the lesion region is required to be divided into at least one ablation region according to the single ablation range of the used ablation catheter.

During the implementation of the ablation, an ablation catheter to be used is required to be determined. In the present embodiment, an effective (definitely killing cells in a region) ablation region generated by once ablation of the ablation catheter is referred to as the single ablation range. The single ablation range is generally defined to be spherical or ellipsoidal, and its shape may be determined by factors such as a relative position of an ablation electrode. In addition, the size of the single ablation range may also be adjusted by controlling parameters, such as power and time, of the ablation.

For a simple case, one ablation region may be used to cover the whole lesion region, as shown in Fig. 5b. A dashed-line circle in Fig. 5b represents the ablation region.

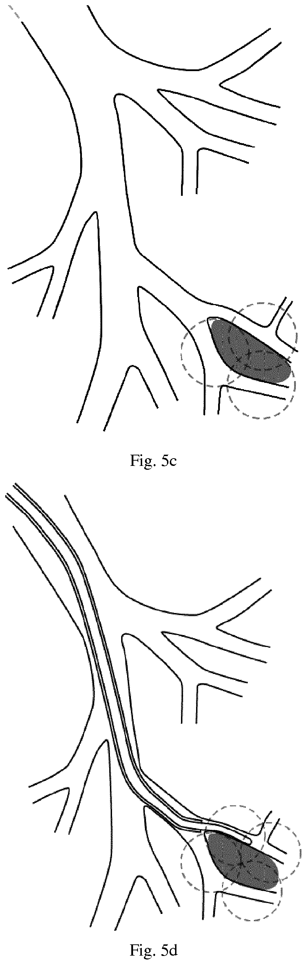

For a complicated case, multiple ablation regions may be used to cover the whole lesion region, as shown in Fig. 5c and Fig. 5d. Each of three dotted-line circles in Fig. 5c represents one ablation region, which means that the ablation is required to be performed for three times. Each of four dotted-line circles in Fig. 5d represents one ablation region, which means that the ablation is required to be performed for four times.

It should be noted that in case of multiple ablation regions, the sizes of different ablation regions may be the same or different.

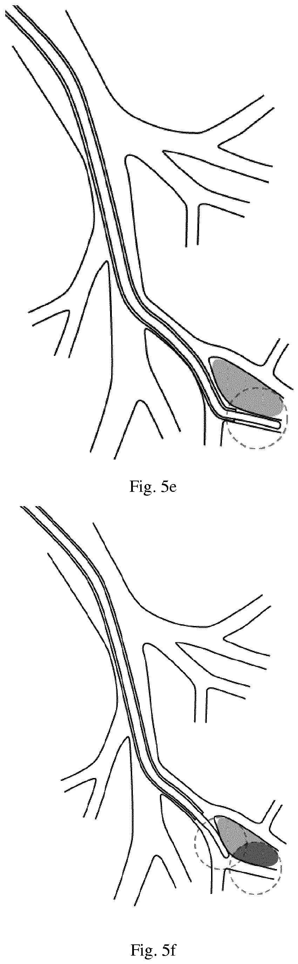

Then, it is necessary to select one of the at least one ablation region to represent a lesion region and the selected ablation region is referred to as the initial ablation region. An ablation process is described by taking the three ablation regions shown in Fig. 5c as examples, and the selected initial ablation region is shown in Fig. 5e. A dotted-line circle in Fig. 5e represents the initial ablation region.

There is no limitation to the manner of selecting the initial ablation region. The initial ablation region may be randomly selected, or the initial ablation region which may be the farthest, or may be the nearest, or may have the widest regional range and the like may be selected.

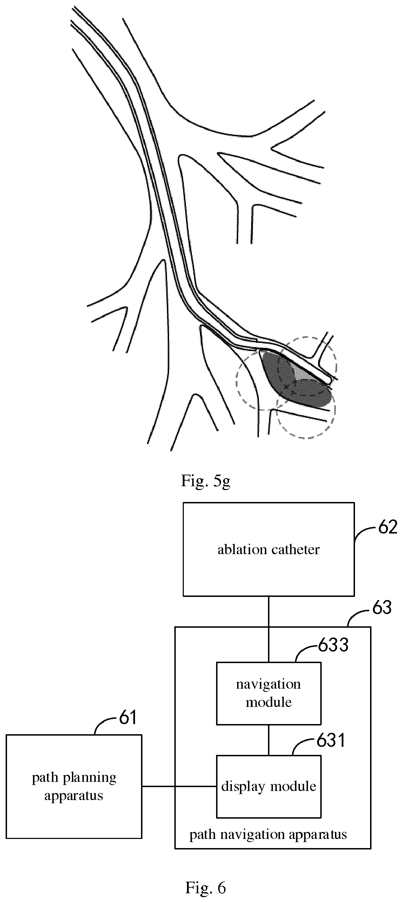

Then, the navigation path to the initial ablation catheter region may be planned on the CT image or the three-dimensional model.
After arriving to the initial ablation region, the ablation catheter may ablate the initial ablation region. After successfully ablating the initial ablation region, the ablation catheter may move from the initial ablation region to the next ablation region, as shown in Fig. 5f, and ablate the ablation region as shown in Fig. 5f. After ablating the ablation region as shown in Fig. 5f, the ablation catheter may continuously move to the next ablation region, as shown in Fig. 5g, to repeatedly perform the ablation until all the ablation regions are ablated. In Fig. 5f and Fig. 5g, dark grey regions represent ablated regions in the lesion region, and light grey regions represent non-ablated regions.

Through division of the lesion region, the medical path planning method provided by the present embodiment is no longer limited by the ellipsoidal shape, so that a planning process and subsequent ablation processes are higher in flexibility and relatively high in efficiency.

It should be noted that executive subjects for all the steps of the methods provided by the above-mentioned embodiments may be the same device, or may be different devices. For example, the executive subject for Step 201 to Step 203 may be a device A. For another example, the executive subject for Steps 201 and 202 may be the device A, and the executive subject for Step 203 may be a device B, etc.

Fig. 6 is a schematic diagram of a structure of a medical path navigation system provided by another embodiment of the present application. As shown in Fig. 6, the system includes: a path planning apparatus 61, an ablation catheter 62 with a localization sensor, and a path navigation apparatus 63.

Herein, the path planning apparatus 61 is configured to plan a navigation path to an initial ablation region on a computed tomography (CT) image of a diseased organ or a 3D reconstruction of the CT scan data, wherein the initial ablation region belongs to one of at least one ablation region included in a lesion region.

The ablation catheter 62 is delivered into an airway leading to the lesion region according to the navigation path to ablate the lesion region.

The path navigation apparatus 63 includes: a display module 631 and a navigation module 633.

Herein, the display module 631 is configured to display the CT image or the three-dimensional model and the navigation path to provide a navigation basis for the navigation module 633.

The navigation module 633 is configured to locate an actual position of the ablation catheter 62 in the airway based on the localization sensor during a moving process of the ablation catheter 62 in the airway, and adjust a position and a direction of the ablation catheter 62 in the airway according to a deviation relation between the actual position of the ablation catheter 62 in the airway and the navigation path, until the ablation catheter 62 is navigated to the initial ablation region.

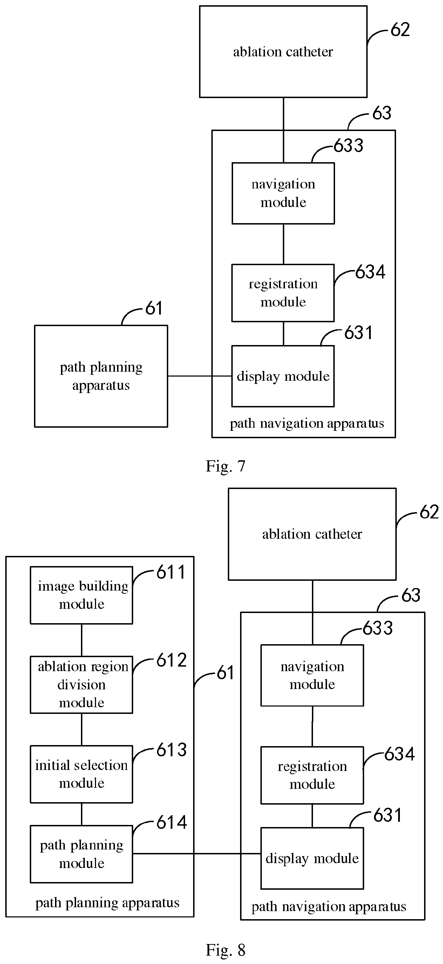

The navigation module 633 is further configured to locate an actual position of the ablation catheter 62 in the lesion region on the basis of the localization sensor during a moving process of the ablation catheter 62 in the lesion region, and adjust a position and a direction of the ablation catheter 62 in the lesion region according to a deviation relation between the actual position of the ablation catheter 62 in the lesion region and other ablation regions except for the initial ablation region in the at least one ablation region, until the ablation catheter 62 is navigated to other ablation regions. In an optional implementation mode, as shown in Fig. 7, the path navigation apparatus 63 further includes: a registration module 634.

The registration module 634 is configured to control the ablation catheter 62 to arrive to a predetermined carina for registration during the moving process of the ablation catheter 62 in the airway before the navigation module 633 navigates the ablation catheter 62, locate an actual position of the carina in the airway based on the localization sensor, and perform coordinate registration on a magnetic field coordinate system where the airway is located and a coordinate system where the CT image or the three-dimensional model is located according to the actual position of the carina in the airway, so as to acquire a mapping relation between the magnetic field coordinate system and the coordinate system where the CT image or the three-dimensional model is located.

Optionally, when adjusting the position and the direction of the ablation catheter 62 in the airway according to the deviation relation, the navigation module 633 is specifically configured to: map the actual position of the ablation catheter in the airway onto the CT image or the three-dimensional model based on the mapping relation between the magnetic field coordinate system and the coordinate system where the CT image or the three-dimensional model is located, and adjust the position and the direction of the ablation catheter in the airway according to the deviation relations between the position as well as the direction, which are displayed on the CT image or the three-dimensional model, of the ablation catheter and the navigation path.

Correspondingly, when adjusting the position and the direction of the ablation catheter 62 in the lesion region, the navigation module 633 is specifically configured to: map the actual position of the ablation catheter in the lesion region onto the CT image or the three-dimensional model based on the mapping relation between the magnetic field coordinate system and the coordinate system where the CT image or the three-dimensional model is located, determine deviation relations between the mapped position as well as the direction of the ablation catheter on the CT image or the three-dimensional model and other ablation regions according to the positions and directions of the ablation catheter displayed on the CT image or the three-dimensional model, and adjust the position and the direction of the ablation catheter in the lesion region according to the deviation relation between the mapped position of the ablation catheter on the CT image or the three-dimensional model and other ablation regions. Optionally, when locating the actual position of the ablation catheter 62 in the airway based on the localization sensor, the navigation module 633 is specifically configured to: calculate the actual position of the ablation catheter 62 in the airway according to an induced current generated by the localization sensor sensing an electromagnetic field at the position of the ablation catheter 62 in the airway.

When locating the actual position of the ablation catheter 62 in the lesion region based on the localization sensor, the navigation module 633 is specifically configured to: calculate the actual position of the ablation catheter 62 in the lesion region according to an induced current generated by the localization sensor sensing an electromagnetic field at the position of the ablation catheter 62 in the lesion region.

In an optional implementation, the localization sensor includes a first localization sensor and a second localization sensor. The first localization sensor is located at the head portion of the ablation catheter 62, and the first localization sensor and the second localization sensor are respectively located on two sides of an ablation electrode on the ablation catheter 62.

Based on the above-mentioned structure of the localization sensor, when locating the actual position of the ablation catheter 62 in the airway based on the localization sensor, the navigation module 633 is specifically configured to: calculate the actual position of the ablation catheter 62 in the airway according to the induced current generated by the first localization sensor and/or the second localization sensor sensing the electromagnetic field at the position of the ablation catheter 62 in the airway. Correspondingly, when locating the actual position of the ablation catheter 62 in the lesion region based on the localization sensor, the navigation module 633 is specifically configured to: calculate the actual position of the ablation catheter 62 in the lesion region according to the induced current generated by the second localization sensor sensing the electromagnetic field at the position of the ablation catheter 62 in the lesion region.

In an optional implementation mode, as shown in Fig. 8, the path planning apparatus 61 includes: an image construction module 611, an ablation region division module 612, an initial selection module 613 and a path planning module 614.

The image construction module 611 is configured to import the CT data of the diseased organ to construct the CT image and the three-dimensional model.

The ablation region division module 612 is configured to determine the lesion region on the diseased organ based on the CT image or the three-dimensional model, and divide the lesion region into at least one ablation region according to a single ablation range of the used ablation catheter.

The initial selection module 613 is configured to select the initial ablation region from the at least one ablation region.

The path planning module 614 is configured to plan a navigation path to the initial ablation region on the CT image or the three-dimensional model.

On the basis of the CT image or the three-dimensional model, the medical path navigation system provided by the present embodiment cooperates with the localization sensor carried by the ablation catheter to perform path navigation in an ablation process. Herein, the ablation catheter may be located to the initial ablation region in the lesion region according to the navigation path planned on basis of the CT image or the three-dimensional model and in combination with the localization sensor. Further, in the lesion region, the ablation catheter is located to other ablation regions in turn based on the localization sensor, so that the problem that the ablation catheter may not be accurately located to a lesion position due to a limitation to the reachable range of a bronchoscope may be solved, and the ablation catheter may be accurately navigated to a lesion position that is not reachable by a bronchoscope, such as lesion positions at the periphery of the lung.

Persons skilled in the art should understand that the embodiments of the present disclosure may provide a method, a system or a computer program product. Therefore, the present disclosure may adopt the form of a complete hardware embodiment, a complete software embodiment, or a software and hardware combined embodiment. In addition, the present disclosure may adopt the form of a computer program product implemented on one or multiple computer available storage media (including, but not limited to, a magnetic disk memory, a Compact Disc Read-Only Memory (CD-ROM), an optical memory and the like) including computer-sensitive program codes.

The present disclosure is described by referring to flowcharts and/or block diagrams of methods, devices (systems) and computer program products according to the embodiments of the present disclosure. It should be understood that each flow and/or each block in the flowcharts and/or the block diagrams and a combination of the flows and/or the blocks in the flowcharts and/or the block diagrams may be implemented by the computer program instructions. These computer program instructions may be provided for a processor of a general computer, a dedicated computer, an embedded processor or other programmable data processing devices to generate a machine, so that an apparatus for achieving functions designated in one or more flows of the flowcharts and/or one or more blocks of the block diagrams is generated via instructions executed by the processor of the computers or the other programmable data processing devices.

These computer program instructions also may be stored in a computer readable memory capable of guiding the computer or other programmable data processing devices to work in a specific manner, so that a manufactured product including an instruction apparatus is generated via the instructions stored in the computer-readable memory, and the instruction apparatus achieves the functions designated in one or multiple flow of flowcharts and/or one or multiple blocks of the block diagrams.

These computer program instructions, which can also be loaded onto the computers or the other programmable data processing devices, enable the computers to implement a series of operation steps on the computers or the other programmable data processing devices; therefore, the instructions executed on the computers or the other programmable data processing devices provide steps of achieving the functions designated in one or multiple flows of the flowcharts and/or one or multiple blocks of the block diagrams.

In a typical configuration, a computing device includes one or multiple Central Processing Units (CPUs), one or multiple input/output interfaces, one or multiple network interfaces and one or multiple internal memories.

The memory may include a non-persistent memory, a Random Access Memory (RAM) and/or a non-volatile memory, etc., such as an ROM or a flash RAM, in a computer-readable medium. The memory is an example of the computer-readable medium.

The computer-readable medium includes persistent, non-persistent, removable media and non-removable media which may realize information storage by any methods or technologies. Information may be computer readable instructions, data structures, modules of programs or other data. Examples of computer storage media include, but are not limited to, a Phase-change Random Access Memory (PRAM), a Static Random Access Memory (SRAM), a Dynamic Random Access Memory (DRAM), other types of RAMs, an ROM, an Electrically Erasable Programmable Read-Only Memory (EEPROM), a flash memory or other memory technologies, a CD-ROM, a Digital Video Disk (DVD) or other optical memories, a magnetic cartridge type magnetic tape, a magnetic tape/disk storage device or other magnetic storage devices or any other non-transmission media, and may be used for storing information accessible by the computing device. The computer readable medium does not include transitory media, such as modulated data signals and carriers, according to definitions herein.

It also should be noted that terms "include", "comprise" or any other variants are meant to cover non-exclusive inclusions, so that a process, method, commodity or device that includes a series of elements not only includes those elements, but also includes other elements which are not definitely listed, or further includes inherent elements of this process, method, commodity or device. Without more restrictions, elements defined by a sentence "includes a/an ..." do not exclude that the process, method, commodity or device that includes the elements still includes other identical elements.

Persons skilled in the art should understand that the embodiments of the present application may provide a method, a system or a computer program product. Therefore, the present application may adopt the form of a complete hardware embodiment, a complete software embodiment, or a software and hardware combined embodiment. In addition, the present application may adopt the form of a computer program product implemented on one or multiple computer-sensitive storage media (including, but not limited to, a magnetic disk memory, a CD-ROM, an optical memory and the like) including computer-sensitive program codes.

The above are merely the embodiments of the present application, but are not intended to limit the present application. Persons skilled in the art can make various changes and modifications to the present application. Any modifications, equivalent replacements, improvements and the like that are made without departing from the spirit and the principle of the present application shall fall within the protection scope defined by the appended claims of the present application.

## Claims

1. A medical path navigation method, comprising:
planning a navigation path to an initial ablation region on a computed tomography (CT) image of a diseased organ or a 3D reconstruction of the CT scan data, wherein the initial ablation region belongs to one of at least one ablation region included in a lesion region;
delivering an ablation catheter with a localization sensor into an airway leading to the lesion region according to the navigation path;
locating an actual position of the ablation catheter in the airway based on the localization sensor during a moving process of the ablation catheter in the airway, and adjusting a position and a direction of the ablation catheter in the airway according to a deviation relation between the actual position of the ablation catheter in the airway and the navigation path, until the ablation catheter is navigated to the initial ablation region; and
locating an actual position of the ablation catheter in the lesion region based on the localization sensor during a moving process of the ablation catheter in the lesion region, and adjusting a position and a direction of the ablation catheter in the lesion region according to a deviation relation between the actual position of the ablation catheter in the lesion region and other ablation regions except for the initial ablation regions in the at least one ablation region, until the ablation catheter is navigated to other ablation regions.

2. The method according to claim 1, wherein prior to navigating the ablation catheter, the method further comprises:
controlling the ablation catheter to arrive to a predetermined carina for registration during the moving process of the ablation catheter in the airway, and locating an actual position of the carina in the airway based on the localization sensor; and
performing coordinate registration on a magnetic field coordinate system where the airway is located and a coordinate system where the CT image or the three-dimensional model is located according to the actual position of the carina in the airway, to acquire a mapping relation between the magnetic field coordinate system and the coordinate system where the CT image or the three-dimensional model is located.

3. The method according to claim 2, wherein the adjusting a position and a direction of the ablation catheter in the airway according to the deviation relation between the actual position of the ablation catheter in the airway and the navigation path comprises:
mapping the actual position and the direction of the ablation catheter in the airway onto the CT image or the three-dimensional model according to the mapping relation between the magnetic field coordinate system and the coordinate system where the CT image or the three-dimensional model is located;
adjusting the position and the direction of the ablation catheter in the airway according to the deviation relation between the position and the direction of the ablation catheter displayed on the CT image or the three-dimensional model, and the navigation path;
correspondingly, the adjusting a position and a direction of the ablation catheter in the lesion region according to the deviation relation between the actual position of the ablation catheter in the lesion region and other ablation regions except for the initial ablation region in the at least one ablation region comprises:
mapping the actual position and the direction of the ablation catheter in the lesion region onto the CT image or the three-dimensional model according to the mapping relation between the magnetic field coordinate system and the coordinate system where the CT image or the three-dimensional model is located;
adjusting the position and the direction of the ablation catheter in the lesion region according to deviation relation between the position and the direction of the ablation catheter displayed on the CT image or the three-dimensional model, and the other ablation regions.

4. The method according to claim 1, wherein the locating an actual position of the ablation catheter in the airway based on the localization sensor comprises:
calculating the actual position of the ablation catheter in the airway according to an induced current generated by the localization sensor sensing an electromagnetic field at the position of the ablation catheter in the airway;
the locating an actual position of the ablation catheter in the lesion region based on the localization sensor comprises:
calculating the actual position of the ablation catheter in the lesion region according to an induced current generated by the localization sensor sensing an electromagnetic field at the position of the ablation catheter in the lesion region.

5. The method according to claim 4, wherein the localization sensor comprises a first localization sensor and a second localization sensor, and the first localization sensor is located at a head portion of the ablation catheter;
the calculating the actual position of the ablation catheter in the airway according to an induced current generated by the localization sensor sensing the electromagnetic field at the position of the ablation catheter in the airway comprises:
calculating the actual position of the ablation catheter in the airway according to the induced current generated by the first localization sensor and/or the second localization sensor sensing the electromagnetic field at the position of the ablation catheter in the airway;
the calculating the actual position of the ablation catheter in the lesion region according to an induced current generated by the localization sensor sensing the electromagnetic field at the position of the ablation catheter in the lesion region comprises:
calculating the actual position of the ablation catheter in the lesion region according to the induced current generated by the second localization sensor sensing the electromagnetic field at the position of the ablation catheter in the lesion region.

6. The method according to claim 1, further comprising:
turning off the localization sensor in an ablation process of the ablation catheter.

7. The method according to any one of claims 1 to 6, wherein prior to planning a navigation path to the initial ablation region on a CT image of a diseased organ or a three-dimensional model reconstructed according to the CT data, the method further comprises:
importing the CT data of the diseased organ to construct the CT image and the three-dimensional model;
determining the lesion region on the diseased organ based on the CT image or the three-dimensional model; and
dividing the lesion region into at least one ablation region according to a single ablation range of the ablation catheter.

8. The method according to claim 7, wherein the lesion region is a single-connected closed three-dimensional region of any shape.

9. A medical path planning method, comprising:
importing computed tomography (CT) data of a diseased organ to construct a CT image and a three-dimensional model;
determining a lesion region on the diseased organ based on the CT image or the three-dimensional model;
dividing the lesion region into at least one ablation region according to a single ablation range of a used ablation catheter;
selecting an initial ablation region from the at least one ablation region; and
planning a navigation path to the initial ablation region on the CT image or the three-dimensional model.

10. The method according to claim 9, wherein the lesion region is a single-connected closed three-dimensional region of any shape.

11. A medical path navigation system, comprising: a path planning apparatus, a path navigation apparatus and an ablation catheter with a localization sensor,
wherein the path planning apparatus is configured to plan a navigation path to an initial ablation region on a computed tomography (CT) image of a diseased organ or a three-dimensional model rebuilt according to CT data, wherein the initial ablation region belongs to one of at least one ablation region included in a lesion region;
the ablation catheter is delivered into an airway leading to the lesion region according to the navigation path to ablate the lesion region;
the path navigation apparatus comprises: a display module and a navigation module;
the display module is configured to display the CT image or the three-dimensional model and the navigation path;
the navigation module is configured to locate an actual position of the ablation catheter in the airway based on the localization sensor during a moving process of the ablation catheter in the airway, and adjust a position and a direction of the ablation catheter in the airway according to a deviation relation between the actual position of the ablation catheter in the airway and the navigation path, until the ablation catheter is navigated to the initial ablation region; and
the navigation module is further configured to locate an actual position of the ablation catheter in the lesion region based on the localization sensor during a moving process of the ablation catheter in the lesion region, and adjust a position and a direction of the ablation catheter in the lesion region according to a deviation relation between the actual position of the ablation catheter in the lesion region and other ablation regions except for the initial ablation region in the at least one ablation region, until the ablation catheter is navigated to other ablation regions.

12. The system according to claim 11, wherein the path navigation apparatus further comprises: a registration module;
the registration module is configured to control the ablation catheter to arrive to a predetermined bulge part for registration during the moving process of the ablation catheter in the airway before the navigation module navigates the ablation catheter, locate an actual position of the bulge part in the airway based on the localization sensor, and perform coordinate registration on a magnetic field coordinate system where the airway is located and a coordinate system where the CT image or the three-dimensional model is located according to the actual position of the bulge part in the airway, to acquire a mapping relation between the magnetic field coordinate system and the coordinate system where the CT image or the three-dimensional model is located.

13. The system according to claim 12, wherein the navigation module is specifically configured to:
map the actual position and the direction of the ablation catheter in the airway onto the CT image or the three-dimensional model based on the mapping relation between the magnetic field coordinate system and the coordinate system where the CT image or the three-dimensional model is located;
adjust the position and the direction of the ablation catheter in the airway according to the deviation relations between the position and the direction of the ablation catheter displayed on the CT image or the three-dimensional model, and the navigation path;
correspondingly, the navigation module is specifically configured to:
map the actual position and the direction of the ablation catheter in the lesion region onto the CT image or the three-dimensional model based on the mapping relation between the magnetic field coordinate system and the coordinate system where the CT image or the three-dimensional model is located;
adjust the position and the direction of the ablation catheter in the lesion region according to the deviation relations between the position and the direction of the ablation catheter displayed on the CT image or the three-dimensional model, and other ablation regions.

14. The system according to any one of claims 11 to 13, wherein,
the navigation module is specifically configured to calculate the actual position of the ablation catheter in the airway according to an induced current generated by the localization sensor sensing an electromagnetic field at the position of the ablation catheter in the airway;
the navigation module is specifically configured to calculate the actual position of the ablation catheter in the lesion region according to an induced current generated by the localization sensor sensing an electromagnetic field at the position of the ablation catheter in the lesion region.

15. The system according to any one of claims 11 to 13, wherein the path planning apparatus comprises:
an image building module, configured to import the CT data of the diseased organ to build the CT image and the three-dimensional model;
an ablation region division module, configured to determine the lesion region on the diseased organ based on the CT image or the three-dimensional model, and divide the lesion region into at least one ablation region according to a single ablation range of the used ablation catheter;
an initial selection module, configured to select the initial ablation region from the at least one ablation region;
a path planning module, configured to plan a navigation path to the initial ablation region on the CT image or the three-dimensional model.
